# EUROPEAN PATENT APPLICATION

(11) **EP 1 736 136 A1**
(43) Date of publication of application: **27.12.2006**
(21) Application number: 06012725.5
(22) Date of filing: 21.06.2006
(51) Int. Cl.: A61K 8/04, A61K 8/64, A61Q 19/00, A61K 9/70, A61K 38/56

(54) **Enzyme inhibiting spray skin barrier compositions**

(30) Priority: 22.06.2005 US 692843 P
(71) Applicant: Bristol-Myers Squibb Company, New York, N.Y. 10154 (US)
(72) Inventor: Sambasivam, Mahesh, Pennington New Jersey 08534 (US); Hoggarth, Andrew, Robert, Crewe Cheshire CW2 YTA (GB); Waring, Mike, Greasby Wirral CH49 1SE (GB); Adam, Fiona, Wrighstown Pennsyilvania 18940 (US)
(74) Representative: Holmes, Miles Keeton

(57) **Abstract**

A skin barrier composition including a barrier component and an enzyme inhibiting component that is sprayable for use in ostomy and wound care, infant diapers, and fecal incontinence applications. The barrier component is based on petrolatum or other acceptable carriers, and the enzyme inhibitor component is derived from tubers, such as potatoes.

## Description

### FIELD OF INVENTION

This invention relates to a sprayable skin barrier composition that acts as a barrier and also inhibits pancreatic and wound enzymes.

### BACKGROUND OF INVENTION

The peristomal, perianal, and perineal skin in subjects with an ostomy or fecal incontinence, and infants wearing diapers can be continuously exposed to feces. When this occurs, the skin is attacked by the contents of feces, mainly the proteolytic or digestive enzymes. This leads to erosion of the skin surface resulting in severe skin conditions, such as dermatitis. Similar skin erosion could also occur in peri-wound skin in a wound environment due to wound enzymes.

In the case of ostomates, a variety of products are used in an attempt to protect the skin from fecal or urine contact. These include film-forming spray barriers, wafers, lotions, creams, pastes, rings, barrier wipes, etc. However, all of these methods of protection only offer a physical barrier to enzymatic attack. When the physical barrier wears out or breaks, the enzymatic attack is imminent.

In the case of a wound, enzymes such as elastase potentially cause skin damage and retard wound healing.

The present invention is related to the protection of the skin by using a spray barrier composition which contains enzyme inhibiting additives in combination with a sprayable barrier.

There are numerous prior art disclosures that describe skin barriers containing ingredients to protect the skin.

U.S. Patent Application No. 2005/0036960A1 discloses a sprayable skin-protectant composition containing a rinse-off resistant agent, a suspending agent, and at least one skin protectant agent, wherein the composition is free of oil and silicone.

U.S. Patent No. 6,723,354 and U.S. Patent Application No. 2004/0166183A1 disclose the use of potato juice in the form of sprays, gel, lotions, powder, etc. for the treatment of inflammation or pruritis. The enzyme inhibition is demonstrated by adding the potato juice directly to a mixture of fecal enzymes.

U.S. Patent No. 6,627,178 B1 and U.S. Patent Application No. 2005/0079229A1 describe systems for applying diaper rash treatment compositions to a selected skin area by forming the composition into a mist or spray using an atomizing spray dispenser.

U.S. Patent Application No. 2005/0048105A1 discusses treatment for diaper rash that include a protease inhibitor such as glycine soja protein or dipalmitoyl hydroxyproline in a polymeric carrier such as Dextran 70 or a copolymer of maleic acid and methylvinyl ether.

U.S. Patent No. 6,331,295 B1 discloses the use of a solid composition for prevention of skin irritation such as diaper rash comprising organophilic clay dispersed in a water-permeable superabsorbent polymer matrix. The organophilic clay is selected from a group consisting of natural and synthetic montmorillonite, bentonite, etc.

U.S. Patent No.6,207,596 B1 discloses a disposable premoistened wipe containing an antimicrobial protease inhibitor such as an aromatic diamidine.

U.S. Patent Application No. 2003/0206944 A1 discloses a wound dressing composed of a cotton cellulose matrix with an active agent that is an inhibitor or sequestrant of a neutrophil-derived cationic protease, such as elastase. The active agent could be inhibitors selected from a group consisting of di- and tri-peptides, or sequestrants selected from a group consisting of sulphonyl, phosphate, or aldehyde groups associated with the matrix.

U.S. Patent No. 6,932,976 discloses the use of a skin protectant cream with an enzyme blocking compound. The enzyme blocking compound is selected from zinc sulfate, zinc chloride, zinc oxide, zinc lactate, or combinations thereof,

U.S. Patent Application No. 2003/0104019A1 discloses a solid topical composition comprising a swellable clay and a peptizing agent for the treatment or reduction of enzymatic dermatitis, such as perineal dermatitis, caused by urine. The swellable clay is selected from a group consisting of pyrophillite, talc, smectite, sepiolite, zeolite, palygorskite, and mixtures thereof. The peptizing agent is selected from a group consisting of tetrasodium or potassium pyrophosphate, sodium hexametaphosphate, sodium citrate, sodium polyacrylate, etc.

These references do not teach how to deliver enzyme inhibiting compositions in a sprayable form or a suitable sprayable skin barrier composition that will inhibit pancreatic and wound enzymes.

### DESCRIPTION OF INVENTION

The present invention relates to incorporating one or more enzyme deactivating agents based on tubers into spray barrier compositions based on oil, silicone, water or petrolatum or a combination thereof.

Accordingly, the spray barrier composition of the present invention includes a barrier component and an enzyme inhibiting component. The enzyme inhibiting component is one or more inhibitors derived from tubers, such as potatoes. The spray barrier component is based on oil, water, silicone, or petrolatum as a carrier.

### Example

Table 1 shows the compositions of the Control Spray Barrier without the potato protein, and Spray Barrier A, with the potato protein. Hexamethyldisiloxane (HMDSO) is a solvent intended to evaporate after spraying. Bis-Ciglyceryl Polyacyladipate-2 is a triglyceride ester of natural vegetable fatty acids. Potato protein under the trade name Protagold FQ was obtained from AVEBE America.

Optionally, other ingredients suitable for skin can be used.

**Table 1. Composition of Control Spray Barrier and Spray Barrier A**

| **Ingredient** | **Control Spray Barrier % (W/W)** | **Spray barrier A % by Weight (W/W)** |
|---|---|---|
| Hexamethyldisiloxane | 50.0 | 48.0 |
| White Petrolatum, USP | 36.9 | 35.3 |
| Bis-Diglyceryl Polyacyladipate-2 | 12.1 | 11.7 |
| Light Mineral Oil, NF | 1.0 | 1.0 |
| Potato Protein | 0.0 | 4.0 |
| Total: | 100.0 | 100 |

The following steps were followed to prepare the above compositions:
(1) Add white petrolatum to mixing vessel and heat to 70°C ± 5°C, with continuous stirring, until material is liquid and free from lumps.
(2) Pre-heat Bis-Diglyceryl Polyacyladipate-2 to 45°C ± 5°C, Add to mixing vessel, with continuous stirring, ensure complete mix with white petrolatum. Reduce temperature to 60°C ± 5°C.
(3) Add light mineral oil to mixing vessel and stir into the formulation.
(4) Add the hexamethyldisiloxane to mixing vessel with continuous stirring and homogenization.
(5) For Spray Barrier A, add the potato protein into the mixing vessel with continuous stirring and homogenization.

The compositions were then packaged into a spray delivery system that provides a mist or spray such as a can-in-can aerosol system. Optionally, the spray barrier formulation can be packaged into a bag-on-valve aerosol system. Both of these systems are known in the art. Essentially, the product is delivered by a system that separates the formulation from the propellant.

Those skilled in the art will realize that there are several known methods of mixing ingredients to obtain a sprayable composition and the procedure described above should not be considered to limit the scope of the present invention.

Both the Control Spray Barrier and Spray Barrier A were found to be sprayable.

### Enzyme Inhibition Assay Study

The enzyme inhibiting capability of Spray Barrier A was compared to the Control Spray Barrier (Control). The Spray Barrier A and the Control were sprayed on to a film and allowed to dry overnight. About 200 mg of the residual material (after solvent evaporation) was used for the assay study in a vial. Table 2 shows the test conditions for the assay study. The vials were incubated at 37°C and the enzyme activity was monitored using absorption-emission spectroscopy.

**Table 2. Test Conditions For Enzyme Inhibition Study**

| **Enzyme** | **Enzyme Concentration, nM** | **pH** | **Buffer** |
|---|---|---|---|
| Chymotrypsin | 0.2 nM | 7.5 | HEPES* |
| Trypsin | 0.25 nM | 7.5 | HEPES* |

| | | | |
|---|---|---|---|
| * N-(2-hydroxyethyl)-piperazine-N'-2-ethanesulfonic acid | | | |

It should be noted that Trypsin and Chymotrypsin are digestive enzymes released by the pancreas.

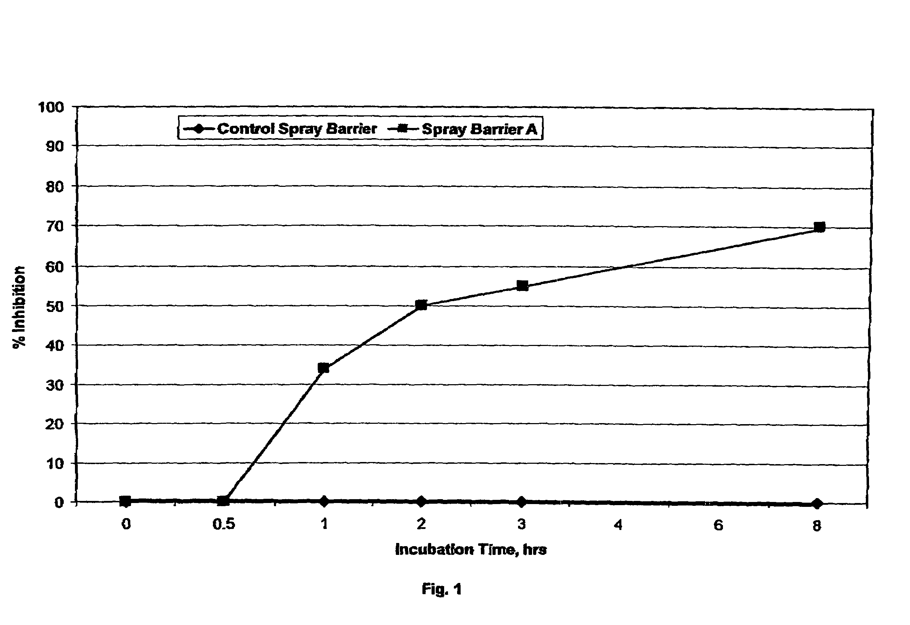

Based on assay studies, the Control Spray Barrier did not show any inhibitory effect on Trypsin and Chymotrypsin, whereas, Spray Barrier A showed inhibitory effect on Chymotrypsin (see Fig. 1) but not on Trypsin at the present concentration of the inhibitor.

It is believed that the Control Spray Barrier will not show inhibitory effect on enzymes present in a wound, such as elastase. However, it is believed that Spray Barrier A with potato protein will show an inhibitory effect on wound enzymes, such as elastase.

## Claims

1. A skin barrier composition comprising a barrier component and an enzyme inhibiting component that is sprayable

2. The skin barrier composition of claim 1, wherein the enzyme inhibiting component is one or more inhibitors derived from tubers.

3. The skin barrier composition of claim 2 wherein the tuber is a potato.

4. The skin barrier composition of claim 1, 2 or 3 wherein the barrier component is based on oil, silicone, or petrolatum or a combination thereof.

5. The skin barrier composition of claim 4 wherein the barrier component is petrolatum.

6. The skin barrier composition of claim 1, 2, 3, 4 or 5 wherein the composition includes hexamethyldisiloxane, white petrolatum, mineral oil, Bis-Diglyceryl Polyacyladipate-2, Aloe Barbadensis Extract, and potato protein.

7. The skin barrier composition of any preceding claim wherein the said composition is used to protect perianal, perineal, peristomal, and peri-wound skin.

8. A skin barrier composition comprising the following;
a. Hexamethyldisiloxane having a percentage by weight from about 20% to about 70.0.%
b. Petrolatum having a percentage by weight from about 1.0% to about 65.0%
c. Bis-Diglyceryl Polyacyladipate-2 having a percentage by weight from about 1.0% to about 65.0%
d. Mineral oil having a percentage by weight from about 0.0% to about 80.0%
e. Aloe Barbadensis Extract having a percentage by weight from about 0.0% to about 20.0%
f. Potato protein having a percentage by weight from about 0.5% to about 30% wherein the said composition is sprayable.
